(19) Europäisches Patentamt
European Patent Office
Office européen des brevets

(11) Publication number: 0 062 772
A2

(12) # EUROPEAN PATENT APPLICATION

(21) Application number: 82101956.9

(22) Date of filing: 11.03.82

(51) Int. Cl.3: C 07 D 249/08
C 07 C 49/233, C 07 C 45/51
C 07 C 49/255, C 07 C 45/71
C 07 D 303/12

(30) Priority: 16.03.81 JP 38307/81

(43) Date of publication of application:
20.10.82 Bulletin 82/42

(84) Designated Contracting States:
BE CH DE FR GB IT LI NL

(71) Applicant: SUMITOMO CHEMICAL COMPANY, LIMITED
15 Kitahama 5-chome Higashi-ku
Osaka-shi Osaka-fu(JP)

(72) Inventor: Tanaka, Shizuya
2-11, Niina-4-chome
Minoo-shi(JP)

(72) Inventor: Funaki, Yuji
10-3-356, Sonehigashinocho-2-chome
Toyonaka-shi(JP)

(72) Inventor: Matsuo, Noritada
29-2, Aza Yamamichi
Minamino Itami-shi(JP)

(74) Representative: PATENTANWÄLTE HENKEL - KERN - FEILER - HÄNZEL
Möhlstrasse 37
D-8000 München 80(DE)

(54) **Regioselective preparation of triazolylvinyl ketones and the intermediates therefor.**

(57) A process for producing triazolylvinyl ketone compounds of the formula (I-E):

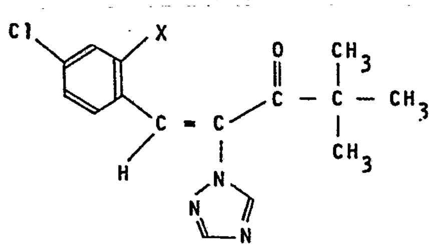

(I-E)

wherein X represents hydrogen atom or chlorine atom, which are useful as fungicide and plant growth regulator, as well as to intermediates used in said process and to a process for producing said intermediates are disclosed.

EP 0 062 772 A2

# REGIOSELECTIVE PREPARATION OF TRIAZOLYLVINYL KETONES AND THE INTERMEDIATES THEREFOR

The present inventoin relates to a process for producing triazolylvinyl ketone compounds of the formula (I-E):

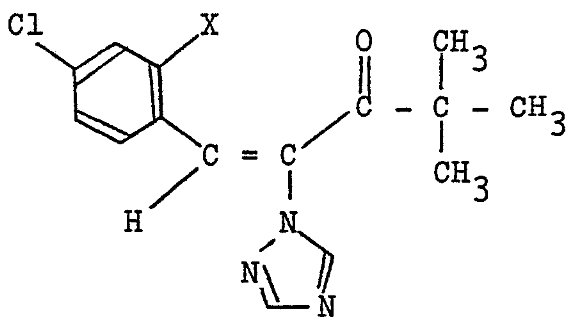

(I-E)

wherein X represents hydrogen atom or chlorine atom, which are useful as fungicides and plant growth regulators, as well as to intermediates used in the process and to a process for producing this intermediates.

Said triazolylvinyl ketone compounds (I-E) are known to be useful as fungicides and plant growth regulators (Published UK Patent Application GB 2046260A).

Since the traizolylvinyl ketone compounds have a double bond in their molecule, they have the following geometric isomers (I-E) and (I-Z):

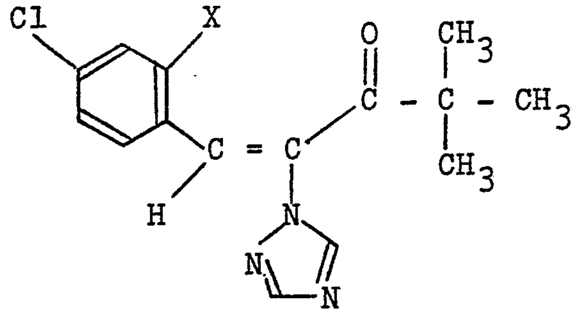

(I-E)

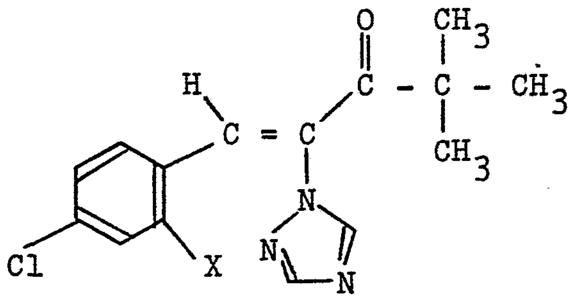

(I-Z)

According to hitherto known techniques, the compound (I-E) has usually been obtained in the form of a mixture with the isomer (I-Z), and purified compound (I-E) has been obtained by irradiating the mixture with light, thereby increasing the content of the compound (I-E), and then, for example, recrystallizing the compound (I-E), or by means of isomerization in the presence of an appropriate isomerization catalyst.

The present inventors conducted various studies on the process for producing triazolylvinyl ketone compounds. As the result, the process of selective production of the isomer (I-E) was invented.

According to the process of the present invention, the triazolylvinyl ketone compound (I-E) is selectively produced by reacting an α-diketone compound of the formula (V):

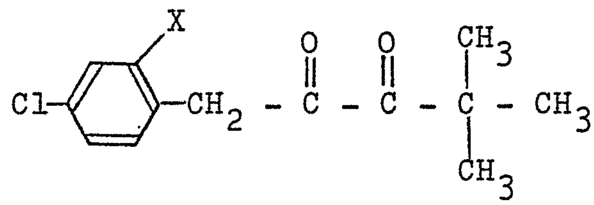 (V)

wherein X represents hydrogen atom or chlorine atom, with thionyl chloride and triazole, thereby a triazolyl group is regioselectively introduced into the compound (V).

Theoretically, there is a possibility of forming two geometric isomers (I-E) and (I-Z) by the introduction of the triazolyl group, but the (I-E) isomer is selectively formed in the process of the present invention and the (I-Z) isomer is not detectable in the product by gas chromatography.

In the published UK Patent Application GB 2046260A, a process for producing (I-E) isomer by irradiating (I-Z) isomer with light is disclosed. The process of the present invention is superior to this known process in that the use of such physical energy and apparatus is unnecessary.

The introduction of imidazolyl group by the use of acetophenone or benzophenone and N,N'-carbonyl-diimidazole or N,N'-thionyl-diimidazole is known (Tetrahedron Letters, No. 52, 5011 (1979); and Heterocycles, 14 (1), 97 (1980)). Neither methods of introducing triazolyl group into carbonyl group nor the stereochemistry of the resulting olefinic products were hitherto known.

Further, the present inventors also discovered an advantageous process for producing the precursors for the above-mentioned production process, including the above-mentioned α-diketone compound.

The production process of the present invention can briefly be expressed by the following scheme:

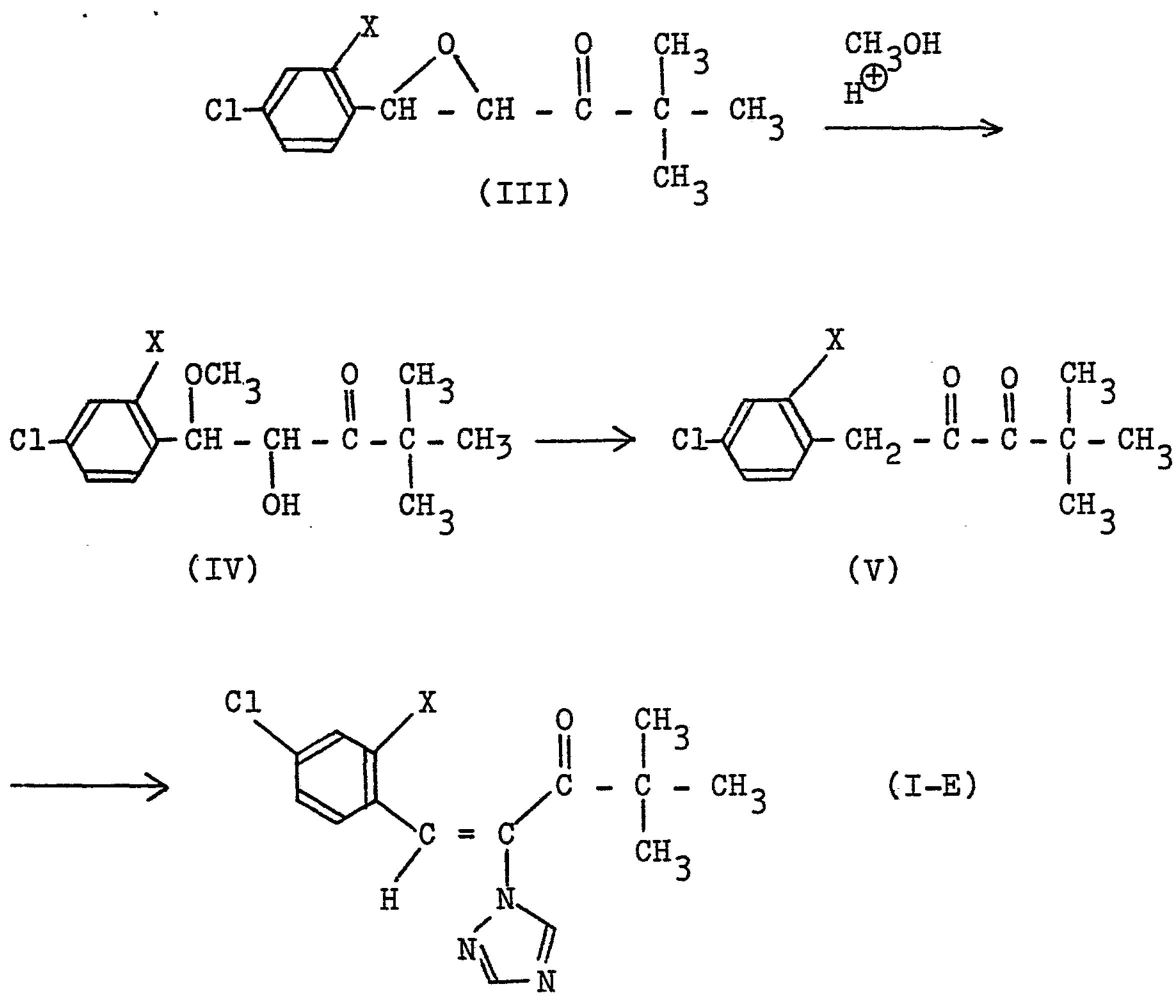


(III) (IV) (V) (I-E)

wherein X is as defined above.

Herein, the intermediate compounds (IV) and (V) are novel compounds.

Thus, the present invention provides a process for producing the triazolylvinyl ketone compounds (I-E),

the intermediates for said production process and a process for producing said intermediates.

The triazolylvinyl ketone compounds (I-E) are useful as fungicides and plant growth regulators. Further, they can be converted to the triazolylvinyl alcohols (II-E) defined below, which are useful as fungicides. Thus, they are also useful as intermediates for the production of the compounds (II-E).

The triazolylvinyl alcohols have a double bond like the triazolylvinyl ketone compounds of the present invention, and accordingly, they have the following two isomers:

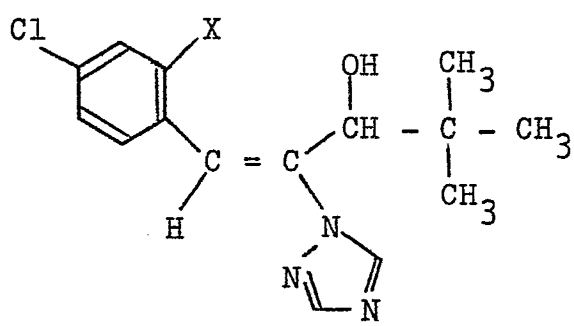

(II-E)

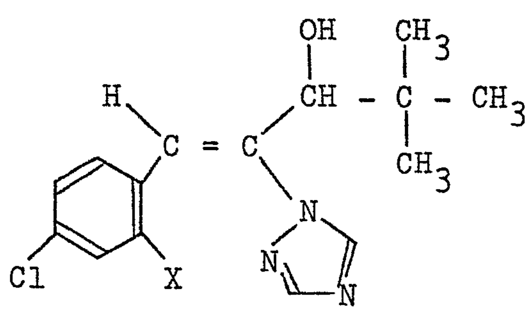

(II-Z)

wherein X is as defined above. Though these isomers are both useful as fungicides, the isomer (II-E) is superior to the isomer (II-Z) in that a smaller amount of application is enough to prevent plants from possible damages by harmful pathogenic fungi and, at the same time, it is effective agaist a wider variety of pathogenic fungi (Published UK Patent Application GB 2046260A).

The triazolylvinyl ketone (I-E) is an important key precursor for the selective production of the triazolylvinyl alcohol (II-E) which is excellent fungicides. Thus, the process of the present invention is of great value also for the production of compounds (II-E).

In carrying out the conversion of the α-diketone (V) to the triazolylvinyl ketone (I-E), 1 mole of the α-diketone compound (V) is reacted with 1-4 moles, preferably 1-3 moles, of thionyl chloride and 4-16 moles, preferably 4-12 moles, of 1,2,4-triazole, half of which is used as dehydrohalogenating agent. It is also possible to carry out the reaction by using 2-8 moles, preferably 2-6 moles, of 1,2,4-triazole and 2-8 moles, preferably 2-6 moles, of a tertiary amine such as trimethylamine, triethylamine, pyridine, picoline, N,N-dimethylaniline, N,N-diethylaniline or the like as dehydrohalogenating agent. It is usually preferable to carry out the reaction in the presence of a solvent. Examples of such solvents are halogenated hydrocarbons such as methylene chloride, chloroform, carbon tetrachloride,

dichloroethane and the like; aromatic hydrocarbons such as benzene, toluene, xylene, chlorobenzene, dichlorobenzene and the like; ethers such as diethyl ether, tetrahydrofuran, dioxane and the like; esters such as ethyl acetate, butyl acetate, ethyl propionate and the like; and nitriles such as acetonitrile, propionitrile and the like. The reaction temperature is in the range of -10°C to +100°C and preferably in the range of 0°C to +70°C. The separation of the product can be achieved either by pouring the reaction mixture into water and extracting the organic layer with a solvent immiscible with water or by filtering off the formed hydrochloride and then concentrating the filtrate. It is also possible to carry out recrystallization or column chromatography in order to increase the purity.

The α-diketone compound of the formula (V) can be produced by reacting a methoxy ketone alcohol compound of the formula (IV) with $PBr_3$. This production can generally be achievable by the use of Lewis acids ($AlCl_3$, $ZnCl_2$, sulfuric acid, phosphorus halides and the like), but phosphorus tribromide is particularly preferable. The reaction is usually carried out in the presence of a solvent. Examples of such solvents are an aromatic hydrocarbon such as benzene or toluene, an aliphatic hydrocarbon such as hexane or heptane, a halogenated hydrocarbon such as chloroform, carbon tetrachloride, chlorobenzene or dichlorobenzene, or an ether such as diethyl ether or tetrahydrofuran. The reaction temperature is in the range of -20°C to +150°C and

preferably in the range of -10°C to +100°C, and usually, 0.1 - 1 mole, preferably 0.3 - 0.5 mole, of phosphorus tribromide is used per 1 mole of the methoxy ketone alcohol (IV). The product is separated by pouring the reaction mixture into water, followed by separating the organic layer or extracting the organic layer with a solvent immiscible with water, then washing the organic layer or the extract with an aqueous alkali solution such as aqueous solution of sodium hydrogen carbonate for the sake of neutralization and finally concentrating the organic layer under reduced pressure.

The methoxy ketone alcohol compound of the formula (IV) can be obtained by reacting the epoxy ketone compound of the formula (III) with methanol in the presence of an acid catalyst.

As the acid catalyst, sulfuric acid, hydrochloric acid, phosphoric acid and the like can be used, and sulfuric acid is particularly preferable. The amount of said Lewis acid used may be in the range of 0.001 - 1 mole per 1 mole of the epoxy ketone compound (III), but a preferable range is 0.1 - 0.5 mole if economy, reaction velocity and easiness of after treatment are taken into consideration. The reaction is usually carried out in an excess amount of methanol which functions as a reagent for the reaction and at the same time as a solvent for the reaction. It is also possible to carry out the reaction in a mixture of methanol and other solvent such as benzene or chloroform.

The reaction temperature is in the range of 10°C to 100°C and preferably in the range of 50°C to the boiling point of reaction mixture (ca. 70°C).

The reaction product is subjected to an after treatment in the usual way. That is, for example, methanol is recovered under reduced pressure to concentrate the reaction mixture, after which water is added and the oily layer is separated or extracted with a solvent immiscible with water.

The epoxy ketone compound (III) can be produced by the following reaction:

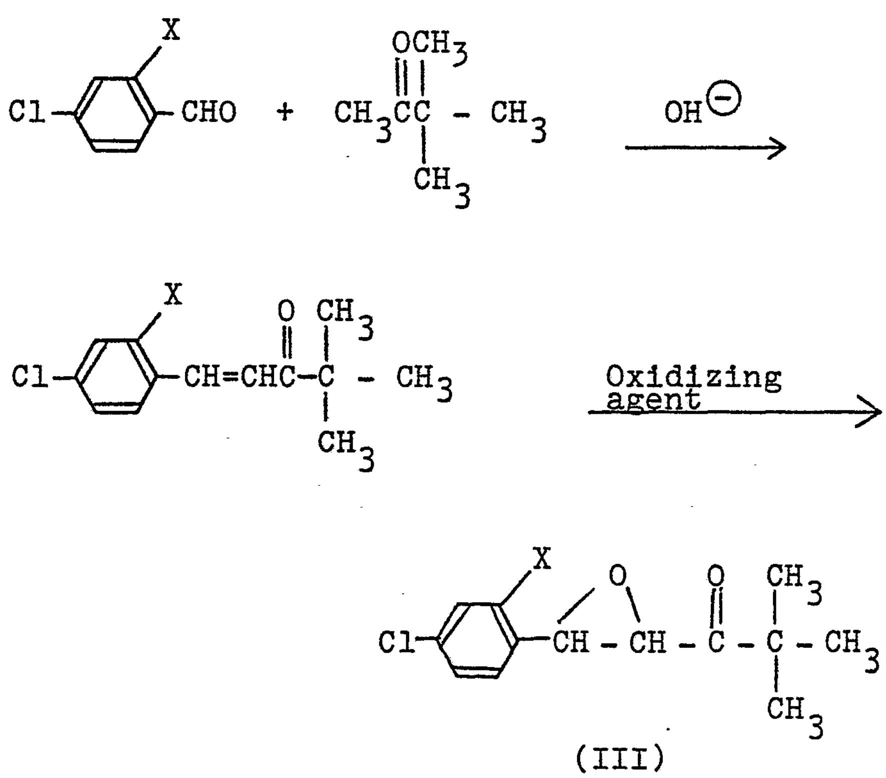


(III)

The first step of the reaction is carried out by using, as a base, sodium hydroxide, potassium hydroxide or the like and in a state diluted with alcohol, aqueous

alcohol or the like. Further information on the reaction is given in Organic Synthesis Col., Vol. I, p. 81 or in the referential example 2 or 3 mentioned later.

The second step of the reaction can be carried out in the usual manner by using oxidizing agent. Examples of such oxidizing agent are aqueous hydrogen peroxide solution, organic peracids, sodium hydrochlorite (NaOCl) and the like. In the case of aqueous hydrogen peroxide solution, the reaction can be effected as mentioned in the referential example 4 or 5, while in the case of organic peracid, it can be achieved by adding meta-chloroperbenzoic acid in chloroform or chlorobenzene. When sodium hydrochlorite is used, the oxidation reaction can be made to progress in the presence of a phase transfer catalyst in a toluene-water mixture system [GAZZ. CHIM. ITAL., 110 (4), 267 (1980)].

The present invention will be explained in more detail with reference to examples. However, the present invention is not limited only to these examples. The NMR spectra are expressed by chemical shifts (δ values) measured by using deutero chloroform as solvent and tetramethylsilane as internal standard.

Example 1 Production of Compound (IV) (X=H)

One milliliter of concentrated sulfuric acid was added to a mixture of 11.9 g (0.05 mole) of 1-(4-chlorophenyl)-4,4-dimethyl-1,2-epoxypentan-3-one and 100 ml of methanol, and the resulting mixture was stirred for one hour. After keeping it at 50°C for 2 hours, it

was concentrated under reduced pressure. After adding water, it was extracted with ethyl acetate. After concentrating the extract under reduced pressure, the residue was recrystallized from carbon tetrachloride/n-hexane (20/1) mixture to obtain 7.93 g (59%) of 1-(4-chlorophenyl)-4,4-dimethyl-2-hydroxy-1-methoxypentan-3-one.

m.p. 119 - 119.5°C

NMR spectrum: 7.26 (4H, s, phenyl proton), 4.53 (1H, broad d, J=7 Hz, proton of methine group having OH), 4.15 (1H, d, J=7 Hz, proton of methine group having $CH_3O$), 3.12 (3H, s, methoxy proton), 2.51 (s, broad s, hydroxy proton), 1.19 (9H, s, t-butyl proton)

Example 2 Production of Compound (IV) (X=Cl)

By the same procedure as in Example 1, 1-(2,4-dichlorophenyl)-4,4-dimethyl-2-hydroxy-1-methoxypentan-3-one was obtained in a quantitative yield. $n_D^{26}$ 1.5159

NMR spectrum: 7.32 (3H, m, phenyl proton), 4.79 (1H, d, J=8 Hz, proton of methine group having $CH_3O$), 4.59 (1H, broad d, J=8 Hz, proton of methine group having OH), 3.12 (3H, s, methoxy proton), 2.88 (1H, broad s, hydroxy proton), 1.18 (9H, s, t-butyl proton)

Example 3 Production of Compound (V) (X=Cl)

Five grams (0.018 mole) of phosphorus tribromide was added at 20°C to a mixture of 14.7 g (0.05

mole) of 1-(4-chlorophenyl)-4,4-dimethyl-2-hydroxyl-1-methoxypentan-3-one and 200 ml of dry diethyl ether. After allowing the resulting mixture to stand for 16 hours, 200 ml of water was added and then sodium hydrogen carbonate was added to alkalify the mixture. After separating the layers, the ether layer was concentrated under reduced pressure and the residue was distilled to obtain 11.1 g (86%) of 1-(4-chlorophenyl)-4,4-dimethyl-pentan-2,3-dione as a yellow colored oily product.

b.p. 78° - 83°C/0.1 mmHg

NMR spectrum: 7.28 (2H, d, J=9 Hz, phenyl proton), 7.06 (2H, d, J=9 Hz, phenyl proton), 3.90 (2H, s, methylene proton), 1.11 (9H, s, t-butyl proton)

Example 4 Production of Compound (V) (X=Cl)

By the same procedure as above, 1-(2,4-dichlorophenyl)-4,4-dimethylpentan-2,3-dione was obtained in a yield of 68%. $n_D^{24}$ 1.5321

NMR spectrum: 7.13 (3H, m, phenyl proton), 4.03 (2H, s, methylene proton), 1.15 (9H, s, t-butyl proton)

Example 5 Production of Compound (I-E) (X=H)

To a solution consisting of 27.7 g (0.4 mole) of triazole and 300 ml of acetonitrile was added 11.9 g (0.1 mole) of thionyl chloride, and the mixture was stirred at 30°C for one hour. After keeping the reaction

mixture at 25°C, 8 g (0.0335 mole) of 1-(4-chlorophenyl)-4,4-dimethylpentan-2,3-dione was dropped thereinto. After 5 hours, the reaction mixture was filtered to remove triazole hydrochloride, and the filtrate was concentrated under reduced pressure. After adding 100 ml of water to the residue, the oily product was extracted with ethyl acetate. By concentrating the extract under reduced pressure, 10.9 g of a yellow-brown colored oily product was obtained. By gas chromatography, any peak corresponding to (I-Z) isomer was not found. By subjecting the product to column chromatography using 200 g of silica gel (n-hexane/acetone = 20/1), 4.34 g (45%) of E-1-(4-chlorophenyl)-4,4-dimethyl-2-(1,2,4-triazol-1-yl)-1-penten-3-one was obtained. Its melting point and NMR spectrum coincided with those of the product obtained by the process of Referential Example 1.

Example 6 Production of Compound (I-E) (X=Cl)

Into 150 ml of acetonitrile was dissolved 16 g (0.23 mole) of triazole at 60°C, into which was dropped 5.6 g (0.047 mole) of thionyl chloride at 40°C. After stirring the mixture at 25°C for 1.5 hours, 50 ml of a solution of 10 g (0.0366 mole) of 1-(2,4-dichlorophenyl)-4,4-dimethylpentan-2,3-dione in acetonitrile was added, and the resulting mixture was stirred in the dark for 20 hours. The insoluble matter was filtered off and the filtrate was concentrated under reduced pressure. Ethyl acetate was added to the residue, the resulting solution

was washed with saturated aqueous solution of sodium hydrogen carbonate and then with water, the organic layer was dried over anhydrous magnesium sulfate, and then it was concentrated under reduced pressure to obtain 11.8 g of an oily product. By purifying it by column chromatography using silica gel, 1.5 g of 1-(2,4-dichlorophenyl)-4,4-dimethylpentan-2,3-dione was recovered and subsequently 0.6 g (5%) of E-1-(2,4-dichlorophenyl)-4,4-dimethyl-2-(1,2,4-triazol-1-yl)-1-penten-3-one (m.p. 92° - 93°C) was obtained. In the gas chromatography, no peak corresponding to (I-Z) was found. Its NMR spectrum coincided with that of the compound mentioned in Japanese Patent Application Kokai (Laid-Open) No. 147,265/80.

Referential Example 1

Production of geometric isomer mixture of 1-(4-chlorophenyl)-4,4-dimethyl-2-(1,2,4-triazol-1-yl)-1-penten-3-one:

A mixture consisting of 50 g of α-(1,2,4-triazol-1-yl)-pinacolone, 41 g of anhydrous potassium carbonate, 200 ml of acetic anhydride and 46.3 g of 4-chlorobenzaldehyde was heated at 90°C for 12 hours with stirring. After cooling the reaction mixture, the precipitate was filtered off. The filtrate was dropped into 500 ml of hot water having a temperature of 60°C to decompose the acetic anhydride. Then it was alkalified by portionwise adding potassium carbonate,

and the resulting oily product was extracted with 500 ml of ethyl acetate. After drying the organic layer on anhydrous sodium sulfate, it was concentrated under reduced pressure. One drop of the residue was taken and made into a solution in acetone, and subjected to gas chromatography under the conditions mentioned below. As the result, a peak corresponding to E-isomer was found at the retention time of 300 sec, and a peak corresponding to Z-isomer was found at the retention time of 360 sec. Their existence ratio calculated from their area percentages was 19.8/61.2, namely about 1/3.

The conditions of the gas chromatography were as follows:

Apparatus: Nippon Denshi 20K, equipped with FID detector;

Column: 1 m Glass column, 5% XE-60, carrier Chromosorb W;

Column temperature: 200°C;

Evaporator temperature: 240°C;

Carrier gas: Nitrogen gas, 1 kg/cm.

The residue was subjected to column chromatography by dissolving it into 100 ml of benzene, passing the solution through a column containing 1.2 kg of silica gel (100-200 mesh) and developing it with n-hexane/acetone (10/1) as a developing solvent. The fractions corresponding to the isomers, thus obtained, were recrystallized from carbon tetrachloride, and there were

obtained 36 g of pure Z-isomer (yield 41.6%, m.p. 78° - 79°C) and 10 g of pure E-isomer (yield 11.5%, m.p. 108° - 109°C). By passing the developing solvent, n-hexane/acetone (10/3) additionally, 8 g of α-(1,2,4-triazol-1-yl)-pinacolone was recovered. Elementary analyses and NMR spectra of the isomers are shown below.

E-isomer of 1-(4-chlorophenyl)-4,4-dimethyl-2-(1,2,4-triazol-1-yl)-1-penten-3-one

Elementary analyses:

| | C (%) | H (%) | N (%) | Cl (%) |
|---|---|---|---|---|
| Calculated (for $C_{15}H_{16}N_3OCl$) | 62.17 | 5.58 | 14.50 | 12.23 |
| Found | 62.32 | 5.60 | 14.41 | 12.20 |

NMR spectrum: 8.11 (1H, s, triazole proton), 7.90 (1H, s, triazole proton), 7.15 (4H, s, phenyl proton), 6.99 (1H, s, olefin proton), 0.99 (9H, s, butyl proton)

Z-isomer of 1-(4-chlorophenyl)-4,4-dimethyl-2-(1,2,4-triazol-1-yl)-1-penten-3-one

Elementary analyses

| | C (%) | H (%) | N (%) | Cl (%) |
|---|---|---|---|---|
| Found | 62.35 | 5.59 | 14.38 | 12.18 |

NMR spectrum: 8.14 (1H, s, triazole proton), 7.98 (1H, s, triazole proton), 7.22 (2H, d, phenyl proton, J=8 Hz), 6.73 (2H, d, phenyl proton, J=8 Hz), 7.49 (1H, s, olefin proton), 1.22 (9H, s, butyl proton).

Referential Example 2

Production of 1-(4-chlorophenyl)-4,4-dimethyl-1-penten-3-one:

To a solution consisting of 2 g (0.05 mole) of sodium hydroxide, 300 ml of 95% ethanol and 50 ml of water were added 70 g (0.5 mole) of 4-chlorobenzaldehyde and 55 g (0.55 mole) of pinacolone, and the mixture was stirred at 25°C for 15 hours. The resulting crystal was collected by filtration, washed with water and a small quantity of ethanol and then dried to obtain 93.5 g (83%) of 1-(4-chlorophenyl)-4,4-dimethyl-1-penten-3-one. m.p. 83° - 84.5°C.

NMR spectrum: 7.45 (1H, d, J=18 Hz, olefin proton), 6.90 (1H, d, J=18 Hz, olefin proton), 7.32 (2H, d, J=8 Hz, phenyl proton), 7.11 (2H, d, J=8 Hz, phenyl proton), 1.21 (9H, s, t-butyl proton).

Referential Example 3

Production of 1-(2,4-dichlorophenyl)-4,4-dimethyl-1-penten-3-one:

By repeating the same procedure as in Referential Example 2, 1-(2,4-dichlorophenyl)-4,4-dimethyl-1-penten-3-one was obtained in a yield of 95%.

m.p. 117.5° - 118.5°C

NMR spectrum: 7.96 (1H, d, J=15 Hz, olefin proton), 7.04 (1H, d, J=15 Hz, olefin proton), 7.42

(3H, m, phenyl proton), 1.23 (9H, s, t-butyl proton).

Referential Example 4

Production of Compound (III) (X=H):

In a solution consisting of 15 g (0.375 mole) of sodium hydroxide, 400 ml of methanol and 50 ml of water was suspended 87.6 g (0.393 mole) of 1-(4-chlorophenyl)-4,4-dimethyl-1-penten-3-one, into which was dropped with stirring 133.6 g of 30% aqueous solution of hydrogen peroxide at 25°C. After 5 hours, the crystalline product was collected by filtration, washed with water and then with a small quantity of methanol, and dried to obtain 69.0 g (74%) of 1-(4-chlorophenyl)-4,4-dimethyl-1,2-epoxypentan-3-one.

m.p. 87° - 88.5°C

NMR spectrum: 7.22 (2H, d, J=9 Hz, phenyl proton), 7.13 (2H, d, J=9 Hz, phenyl proton), 3.80 (2H, s, epoxymethine proton), 1.21 (9H, s, t-butyl proton).

Referential Example 5

Production of Compound (III) (X=Cl):

By the same procedure as in Referential Example 4, 1-(2,4-dichlorophenyl)-4,4-dimethyl-1,2-epoxypentan-3-one was obtained in a yield of 55%.

m.p. 60° - 61°C

NMR spectrum: 7.23 (3H, m, phenyl proton), 4.12

(1H, d, J=2 Hz, epoxymethine proton), 3.67 (1H, d, J=2 Hz, epoxymethine proton), 1.25 (9H, s, t-butyl proton).

WHAT IS CLAIMED IS:

1. A process for producing E-isomer of triazolylvinyl ketone represented by the formula,

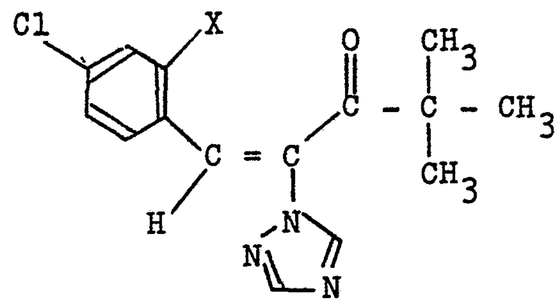

wherein X represents hydrogen atom or chlorine atom, which comprises reacting an α-diketone compound of the formula,

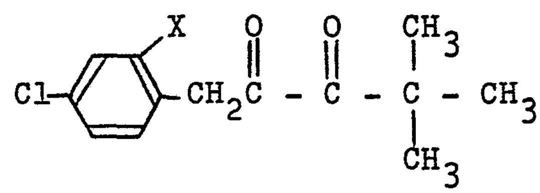

,

wherein X is as defined above, with thionyl chloride and 1,2,4-triazole.

2. A process according to Claim 1, wherein the reaction is carried out in the presence of a tertiary amine as a dehydrohalogenating agent selected from the group consisting of trimethylamine, triethylamine, pyridine, picoline, N,N-dimethylaniline and N,N-diethylaniline and a solvent selected from the group consisting of a halogenated hydrocarbon, an aromatic hydrocarbon, an ether, an ester, and a nitrile.

3. An α-diketone compound of the formula,

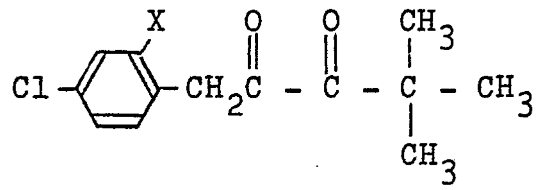

wherein X represents hydrogen atom or chlorine atom.

4. A process for producing an α-diketone compound of the formula,

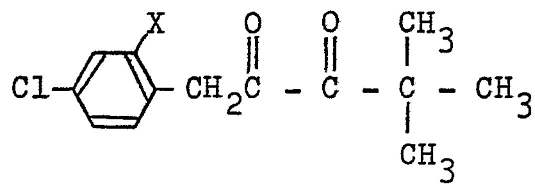

wherein X represents hydrogen atom or chlorine atom, which comprises reacting a methoxy ketone alcohol compound of the formula,

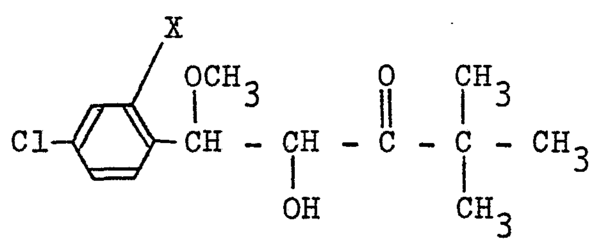

wherein X is as defined above, with a Lewis acid.

5. A process according to Claim 4, wherein the reaction is carried out in the presence of a solvent selected from the group consisting of an aromatic hydrocarbon, an aliphatic hydrocarbon, a halogenated hydrocarbon and an ether.

6. A process according to Claim 4, wherein said Lewis acid is phosphorus tribromide.

7. A methoxy ketone alcohol compound of the formula,

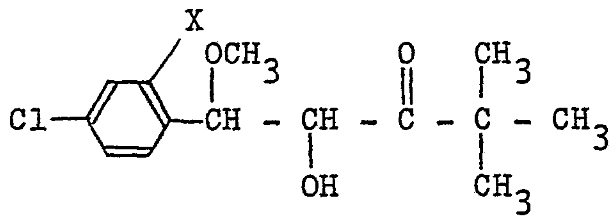

wherein X represents hydrogen atom or chlorine atom.

8. A process for producing a methoxy ketone alcohol compound represented by the formula,

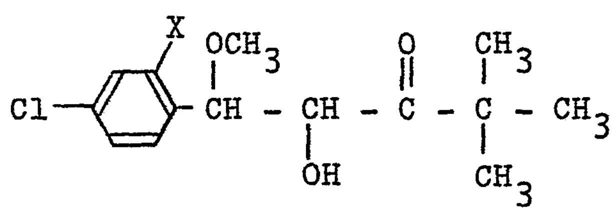

wherein X represents hydrogen atom or chlorine atom, which comprises reacting an epoxy ketone compound represented by the formula,

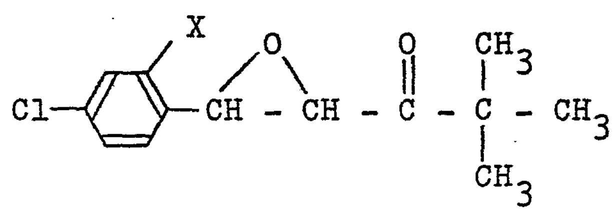

wherein X is as defined above, with methanol.

9. A process according to Claim 8, wherein the reaction is carried out in the presence of an acid catalyst selected from the group consisting of sulfuric acid, hydrochloric acid and phosphoric acid and an excess amount of methanol.